# EUROPÄISCHE PATENTSCHRIFT

(11) **EP 0 944 368 B1**
(45) Veröffentlichungstag und Bekanntmachung des Hinweises auf die Patenterteilung: **05.02.2003**
(21) Anmeldenummer: 97952792.6
(22) Anmeldetag: 21.11.1997
(51) Int. Cl.: A61F 2/34

(54) **GELENKPFANNE FUR EINE HÜFTGELENKENDOPROTHESE**
ARTIFICIAL ACETABULAR CUP
COTYLE SYNTHETIQUE

(30) Priorität: 21.11.1996 DE 19648263; 20.01.1997 DE 19701778
(43) Veröffentlichungstag der Anmeldung: 29.09.1999
(73) Patentinhaber: PLUS ENDOPROTHETIK AG, 6343 Rotkreuz (CH)
(72) Erfinder: HORBER, Willi, CH-8005 Zürich (CH)
(74) Vertreter: Popp, Eugen, Dr.
(86) Internationale Anmeldenummer: EP9706515
(87) Internationale Veröffentlichungsnummer: WO98022049

(56) Entgegenhaltungen:
- EP-A- 0 303 006
- EP-A- 0 380 045
- EP-A- 0 552 949
- EP-A- 0 601 224
- EP-A- 0 612 509
- EP-A- 0 648 478
- WO-A-92/15261
- DE-A- 3 205 526
- DE-A- 4 211 347
- DE-A- 4 337 936
- DE-A- 4 442 559
- FR-A- 2 715 556
- US-A- 5 176 711
- US-A- 5 326 368
- US-A- 5 571 198

## Beschreibung

Die Erfindung betrifft eine Gelenkpfanne für eine Hüftgelenkendoprothese, mit einem länglich geformten Pfannenkörper und einem Pfanneneinsatz (Inlay), der auf der Frontseite eine halbkugelige Höhlung zur Aufnahme einer Gelenkkugel aufweist. Eine solche gelenkpfanne ist z.B. ans der Druckschrift WO-A-92 15 261 bekannt.

Endoprothesen für den Ersatz des Hüftgelenks sind seit langer Zeit bekannt. Es wird dazu auf die DE-U 72 40 856 oder DE-A 26 11 985 verwiesen. In der DE-A 23 01 810 wird eine Gelenkpfanne beschrieben, die aus einer Außen- und einer Innenkappe besteht, wobei die Innenkappe lösbar in der Außenkappe untergebracht ist und ihrerseits eine Gelenkkugel aufnimmt. In der EP-B 0 303 006 ist eine Revisionspfanne beschrieben, die also insbesondere als Ersatz für eine implantierte Gelenkpfanne bei ausgearbeitetem Pfannendach des Acetabulums geeignet ist. Diese Gelenkpfanne umfaßt einen Pfannenkörper, der auf der Frontseite eine halbkugelige Höhlung zur Aufnahme einer Gelenkkugel aufweist, wobei die Achse der Höhlung außermittig des Pfannenkörpers liegt und wobei der Pfannenkörper im Schnitt senkrecht zur außermittig liegenden Achse der Höhlung länglich oval geformt ist. Die bekannte Gelenkpfanne sieht also einen länglich geformten Pfannenkörper vor, der sowohl in Längsrichtung als auch in Richtung quer dazu symmetrisch ausgebildet ist, nämlich annähernd ein Oval bildet. Die halbkugelige Höhlung liegt vorzugsweise in dem Bereich, in dem die anatomische Höhlung im Acetabulum gelegen hat. Der Sinn dieser Konstruktion liegt darin, daß sich die Gelenkpfanne stark der Anatomie annähert, wie sie beim Abarbeiten des Pfannendachs durch eine gelockerte prothetische Gelenkpfanne entsteht. Dementsprechend muß nur noch minimale Knochensubstanz entfernt oder ersetzt werden, um die Gelenkpfanne einzusetzen.

Machteilig beim Stand der Technik ist jedoch die symmetrische Ausbildung der Gelenkpfanne mit der Folge, daß in der Regel nur eine sogenannte Zwei-Punkt-Verspannung derselben innerhalb des Acetabulums erreichbar ist. Um die Verbindungslinie zwischen diesen beiden Punkten werden Kippmomente wirksam, die nach längerer Implantationszeit zu einer Lockerung der Pfanne führen können. Die Gelenkpfanne muß dann erneut ausgetauscht werden.

Der vorliegenden Erfindung liegt die Aufgabe zugrunde, eine Gelenkpfanne der eingangs genannten Art zu schaffen, die eine statisch bestimmte Verspannung innerhalb des Acetabulums erlaubt, so daß keine Kippmomente wirksam werden können, die zu einer Lockerung der Pfanne führen. Vor allem gilt es, eine definierte Mehrpunkt-Verspannung zu erhalten.

Diese Aufgabe wird erfindungsgemäß durch die kennzeichnenden Merkmale des Anspruches 1 gelöst, wobei vorteilhafte Weiterbildungen und konstruktive Details in den Unteransprüchen beschrieben sind. Diesbezüglich wird insbesondere auf Anspruch 2 verwiesen, wonach der Pfannenkörper in Frontansicht unter anderem einen nieren- oder bohnenförmigen Umriß aufweist. Dieser Umriß führt zu einer Drei-Punkt-Verspannung an den konvexen Extrempunkten. Diese Verspannung ist statisch bestimmt und dementsprechend stabil.

Alternativ könnte der Umriß des Pfannenkörpers trapezförmig, dreieckförmig, halbmondförmig, herzförmig, pfeilförmig, halbkreisförmig oder dergleichen asymmetrisch ausgebildet sein. Es muß lediglich sichergestellt sein, daß eine statisch bestimmte Mehrpunkt-Verspannung erhalten wird, um'eine stabile Implantation zu erreichen.

Von ganz besonderem Vorteil ist auch noch die Ausführungsform nach Anspruch 6, wonach der Pfanneneinsatz innerhalb einer korrespondierenden Ausnehmung im Pfannenkörper fixierbar ist, und zwar in unterschiedlichen Winkelstellungen relativ zur Mittenachse der erwähnten Ausnehmung, wobei die Ausnehmung vorzugsweise konzentrisch im Pfannenkörper ausgebildet ist. Bei außermittig angeordneter halbkugeliger Höhlung im Pfanneneinsatz läßt sich dann diese Höhlung in eine gewünschte Position relativ zum Pfannenkörper bringen. Somit läßt sich nicht nur die kranio/kaudale Lage der halbkugeligen Höhlung, sondern auch die medio/laterale Lage derselben variieren. Dies ist dann von Vorteil, wenn wegen Knochendefekten die Pfanne nicht in Idealposition implantiert werden kann.

Des weiteren sei noch gesondert erwähnt die Ausführungsform nach den Ansprüchen 9 und 10, wonach die Frontseite des pfannenkörpers im kranialen Bereich nach vorne überdacht ist, während sie im kaudalen Bereich nach innen bzw. hinten zurückgenommen ist. Durch die Überdachung im kranialen Bereich wird bei Steilstellung der Pfanne eine Luxation der Gelenkkugel verhindert. Die Rücknahme im kaudalen Bereich ermöglich in einem solchen Fall auch eine ausreichende Adduktion des Beines.

Von Vorteil ist auch noch, wenn der Boden des Pfannenkörpers einen oder mehrere Durchgänge aufweist, der bzw. die durch einen Deckel, insbesondere verschiebbar oder drehbar gelagerten Deckel verschließbar ist bzw. sind. Damit ist es möglich, nach Einsetzen des Pfannenkörpers im Becken des Patienten die Setztiefe zu kontrollieren und Spongiosa zwischen Knochen und dem Boden des Pfannenkörpers einzubringen. Um einen Kontakt zwischen dem Pfanneneinsatz bzw. Inlay, welches vorzugsweise aus Kunststoff besteht, und dem Knochen zu verhindern, wird der erwähnte Durchgang im Boden des Pfannenkörpers mit einem Deckel verschlossen, und zwar vorzugsweise einem drehbar gelagerten Deckel. Eine alternative Lösung sieht vor, die Rück- bzw. Außenseite des Pfanneneinsatzes aus dem gleichen Material oder einem ähnlichen Material wie den Pfannenkörper und dergestalt zu formen, daß beim Einsetzen des Pfanneneinsatzes in den Pfannenkörper die Rückseite des Pfanneneinsatzes einen oder mehrere Durchgänge im Boden des Pfannenkörpers wie ein Deckel verschließt. Diese Lösung ist vor allem handhabungstechnisch aber auch herstellungstechnisch sehr vorteilhaft.

Um die Fixierung des Pfannenkörpers zusätzlich zu erhöhen, kann dieser an der Außenseite mehrere etwa gleichmäßig über den Umfang verteilt angeordnete Einschlagrippen mit messerartigen Schneiden aufweisen, wobei sich die Einschlagrippen etwa parallel zur Mittenachse des Pfannenkörpers erstrecken.

Des weiteren kann zu diesem Zweck der Pfannenkörper im kranialen und/oder kaudalen Bereich Löcher für den Durchtritt von Knochenschrauben aufweisen, wobei die Schraubenlöcher vorzugsweise sanduhr- bzw. venturirohrartig ausgebildet sind, so daß die Schrauben zwängungsfrei unter unterschiedlichen Winkeln eingeschraubt werden können. Auch können Stopfelemente vorgesehen werden, um unbenutzte Schraubenlöcher zu verschließen. Die Schraubenlöcher befinden sich vorzugsweise zwischen den erwähnten Einschlagrippen des Pfannenkörpers.

Die Fixierung des Pfanneneinsatzes in der korrespondierenden Ausnehmung des Pfannenkörpers erfolgt vorteilhafterweise durch einen Schnappmechanismus. Denkbar ist es, zu diesem Zweck an der Außenseite des Pfanneneinsatzes einen umlaufenden Wulst vorzusehen, der in eine korrespondierende Ringnut innerhalb der Ausnehmung des Pfannenkörpers einrastet.

Die Oberfläche der im Pfanneneinsatz ausgebildeten halbkugeligen Höhlung ist vorzugsweise mit einer Gleitschicht versehen, insbesondere einer Gleitschicht aus Metall, Keramik oder einem abriebfesten Kunststoff. Der Pfanneneinsatz kann auch insgesamt aus einem der vorgenannten Materialien, insbesondere Keramik oder auch einer Kombination dieser Materialien hergestellt sein.

Zur Fixierung des Pfanneneinsatzes innerhalb der korrespondierenden Ausnehmung im Pfannenkörper unter einem vorbestimmten Winkel relativ zur Mittenachse der erwähnten Ausnehmung kann der Pfanneneinsatz randseitig wenigstens einen sich radial nach außen erstreckenden Vorsprung oder alternativ wenigstens eine Vertiefung aufweisen, der bzw. die mit wenigstens einer in der Ausnehmung des Pfannenkörpers ausgebildeten Vertiefung bzw. einem in der Ausnehmung des Pfannenkörpers ausgebildeten Vorsprung korrespondiert. Vorzugsweise sind mehrere gleichmäßig über den Umfang verteilt angeordnete Vorsprünge bzw. Vertiefungen ausgebildet.

Nachstehend wird eine bevorzugte Ausführungsform einer erfindungsgemäß ausgebildeten Gelenkpfanne anhand der beigefügten Zeichnung näher erläutert. Es zeigen:
- Figur 1: einen erfindungsgemäß ausgebildeten Pfannenkörper in Seitenansicht;
- Figur 2: einen erfindungsgemäß ausgebildeten Pfannenkörper in Draufsicht;
- Figur 3: den Pfannenkörper gemäß Figur 2 im Schnitt Längslinie A-A in Figur 2;
- Figur 4: den Pfannenkörper gemäß den Figuren 1 bis 3 in perspektivischer Ansicht von schräg oben;
- Figur 5: einen Pfanneneinsatz (Inlay) für den Pfannenkörper gemäß den Figuren 1 bis 4 in perspektivischer Ansicht von schräg oben;
- Figur 6: Unteransicht des Pfanneneinsatz gemäß Figur 5;
- Figur 7: den Pfanneneinsatz gemäß Figur 6 in Seitenansicht;
- Figur 8: den Pfanneneinsatz gemäß Figur 6 im Schnitt Längslinie A-A in Figur 6; und
- Figur 9: einen Pfanneneinsatz mit angeformten Deckel im Schnitt.

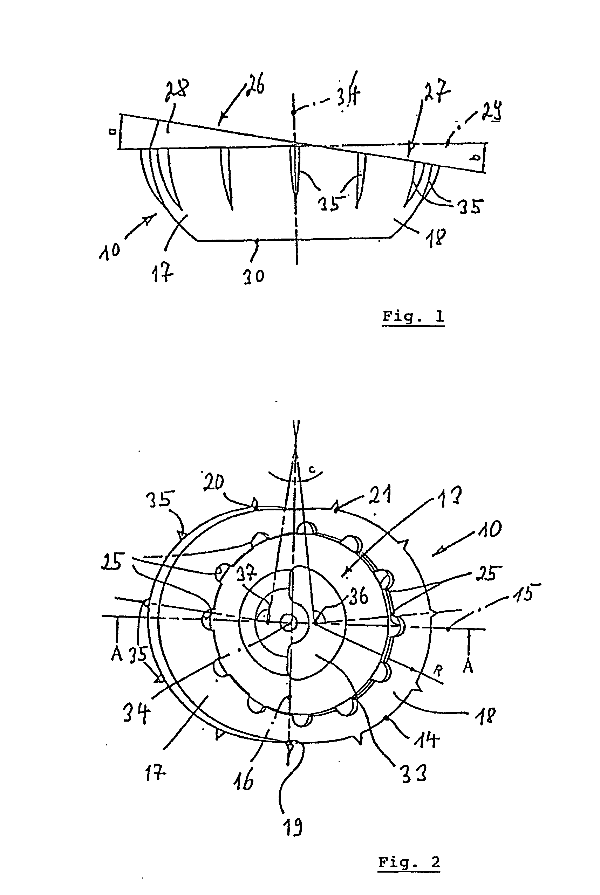

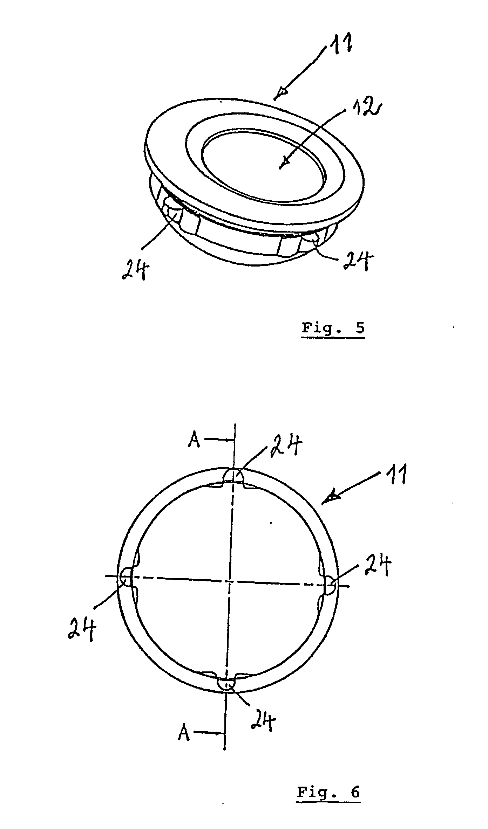

In den Figuren 1 bis 4 ist ein länglich geformter Pfannenkörper 10 einer Gelenkpfanne für eine Hüftgelenkendoprothese dargestellt. Die Figuren 5 bis 8 zeigen einen rotationssymmetrischen Pfanneneinsatz 11, der auf der Frontseite eine halbkugelige Höhlung 12 zur Aufnahme einer nicht dargestellten Gelenkkugel aufweist. Der Pfanneneinsatz 11 ist zur Aufnahme innerhalb einer korrespondierenden Ausnehmung 13 im Pfannenkörper 10 bestimmt.

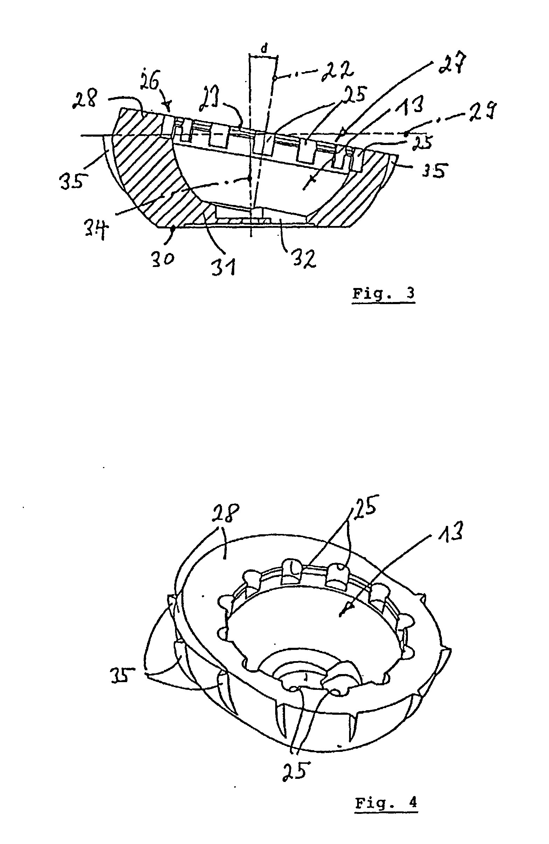

Wie Figur 2 sehr gut erkennen läßt, ist der Pfannenkörper 10 in Front- bzw. Draufsicht länglich geformt, wobei der Umriß 14 in bezug auf die Längsachse 15 asymmetrisch ausgebildet ist. Konkret ist bei der dargestellten Ausführungsform der Umriß des Pfannenkörpers 10 in Front- bzw. Draufsicht nieren- bzw. bohnenförmig ausgebildet. Dieser Umriß wird durch Verbindung bzw. Überbrückung von zwei zueinander im Winkel "c" relativ zur Querachse 16 des Pfannenkörpers 10 gegenüberliegenden Viertelkugelschalen 17 und 18 gebildet, wobei diese Viertelkugelschalen entsprechend den Figuren 1 und 3 bodenseitig abgeflacht sind. Es sei an dieser Stelle darauf hingewiesen, daß der Pfannenkörper statt durch Viertelkugelschalen auch definiert sein kann durch die beschriebene zusammenfügung von Hälften oder Teilabschnitten zweier Rotationskörper wie Kugelsegmente, Kugelhauben, Kegel, Zylinder oder dergl. oder auch prismatischer Körper. Bei solchen Formkörpern treten dann anstelle der Kugelmittelpunkte die Rotations- bzw. Symmetrieachsen, die im wesentlichen parallel zu der weiter unten noch erwähnten Mittenachse 34 liegen.

Die den Viertelkugelschalen zugeordneten Kugelzentren weisen außerdem einen Abstand voneinander auf. Der Pfannenkörper 10 ist somit zwar in bezug auf die Querachse 16 symmetrisch, nicht jedoch in bezug auf die Längsachse 15. Dadurch wird eine sogenannte Drei-Punkt-Verspannung innerhalb des Acetabulums erreicht, und zwar an den konvexen Extrempunkten, die in Figur 2 mit den Bezugsziffern 19, 20 und 21 gekennzeichnet sind. Auf diese Weise ist eine dauerhaft stabile Implantation des Pfannenkörpers 10 innerhalb des entsprechend ausgearbeiteten Acetabulums sichergestellt, und zwar insbesondere zementfreie Implantation.

Der Pfannenkörper besteht aus einem körperverträglichen Metall, beispielsweise einer Kobaltchrommolybdänlegierung, Titan- oder Titanlegierung.

Der in den Figuren 5 bis 8 dargestellte Pfanneneinsatz 11 ist vorzugsweise aus einem körperverträglichen Material wie Metall, Keramik oder Kunststoff, insbesondere Polyethylen oder dergleichen, allein oder in Kombination miteinander, hergestellt.

Der Pfanneneinsatz 11 ist innerhalb der bereits erwähnten Ausnehmung 13 im Pfannenkörper 10 fixierbar, und zwar bei der dargestellten Ausführungsform in unterschiedlichen Winkelstellungen relativ zur Mittenachse 22 der Ausnehmung 13, wobei diese Ausnehmung 13 vorzugsweise konzentrisch im Pfannenkörper 10 ausgebildet ist. In Abweichung davon ist die Ausnehmung 13 bei der dargestellten Ausführungsform nach kaudal hin versetzt. Des weiteren ist die Eingangsebene der Ausnehmung 13 in Richtung von kranial nach kaudal um den Winkel "d" geneigt.

Der Pfanneneinsatz 11 ist rotationssymmetrisch ausgebildet, und zwar konkret halbkugelförmig. Er ist kraft- und formschlüssig innerhalb der korrespondierenden Ausnehmung 13 im Pfannenkörper 10 fixierbar. Zu diesem Zweck weist der Pfanneneinsatz 11 randseitig vier gleichmäßig über den Umfang verteilt angeordnete, sich radial nach außen erstreckende Vorsprünge 24 auf, die mit einer Vielzahl gleichmäßig über den Umfang verteilt und diametral zueinander angeordneten Vertiefungen 25 in der Ausnehmung 13 des Pfannenkörpers 10 korrespondieren derart, daß die Winkelstellung des Pfanneneinsatzes 11 frei wählbar ist. Ferner ist die halbkugelige Höhlung 12 des Pfanneneinsatzes 11 exzentrisch angeordnet, und zwar dergestalt, daß nach Einsetzen des Pfanneneinsatzes in den Pfannenkörper der Kugelmittelpunkt der halbkugeligen. Höhlung 12 je nach Winkelstellung des Pfanneneinsatzes exakt in Höhe der Querachse 16 des Pfannenkörpers 10 zu liegen kommt (Winkelstellung 0 Grad) oder aber kaudal dazu, wobei der maximal kaudale Abstand bei einer Winkelstellung von 180 Grad erreicht wird. Durch Variieren der Winkelstellung läßt sich aber nicht nur die kranio/kaudale Lage des Kugelzentrums wählen, sondern auch die medio/laterale Lage in bezug auf die Längsachse 15 des Pfannenkörpers 10. Dies ist dann von Vorteil, wenn wegen Knochendefekten die Pfanne nicht in Idealposition implantiert werden kann.

Entsprechend den Figuren 1 und 3 ist die Frontseite des Pfannenkörpers 10 im kranialen Bereich 26 nach vorne überdacht (Überdachung 28). Im kaudalen Bereich 27 ist die Frontseite des Pfannenkörpers 10 nach innen bzw. hinten zurückgenommen. Die Überdachung 28 erfolgt unter einem Winkel "a", während die kaudale Rücknahme unter einem Winkel "b" durchgeführt ist. Diese Winkel sind angegeben in bezug auf eine Hauptpfannenebene 29, die sich parallel zur Abflachung 30 des Bodens des Pfannenkörpers 10 erstreckt. Die Winkel "a" und "b" können gleich oder unterschiedlich groß sein. Es ist auch denkbar, nur eine Überdachung und keine Rücknahme, oder nur eine Rücknahme und keine Überdachung vorzusehen.

Die formschlüssige Fixierung des Pfanneneinsatzes 11 innerhalb der Ausnehmung 13 des Pfannenkörpers 10 mittels der am Umfangsrand des Pfanneneinsatzes 11 angeordneten Vorsprünge bzw. Noppen 24 einerseits und den korrespondierenden, am frontseitigen Umfangsrand der Ausnehmung 13 ausgebildeten Vertiefungen 25 andererseits kann ersetzt werden durch eine rein kraftschlüssige Fixierung ohne Vorsprünge und Vertiefungen. Diese rein kraftschlüssige Fixierung erlaubt eine beliebige Winkelstellung des Pfanneneinsatzes 11 innerhalb der Ausnehmung 13 im Pfannenkörper 10.

Der Boden 31 des Pfannenkörpers 10 weist einen Durchgang 32 auf, der durch einen Deckel 33 verschließbar ist. Der Deckel 33 ist um eine sich senkrecht zum Boden 31 erstreckende Achse 34 drehbar gelagert. Die Bodenöffnung 32 erstreckt sich nur über einen vorbestimmten Kreissektor, der durch einen entsprechend sektoriellen Deckel verschließbar ist. Der Deckel 33 ist als sektorielle Lamellenscheibe ausgebildet.

Der Durchgang 32 ermöglicht es, nach Einsetzen des Pfannenkörpers 10 im Acetabulum die Setztiefe zu kontrollieren und/oder Spongiosa zwischen dem Boden des Pfannenkörpers 10 und dem Knochen einzubringen. Anschließend wird der Durchgang 32 mittels des Deckels 33 verschlossen, so daß der Pfanneneinsatz 11 nicht in Berührung mit dem Knochen kommt.

Entsprechend den Figuren 1, 2 und 4 weist der Pfannenkörper 10 außenseitig sich parallel zur Mittenachse 34 erstreckende Einschlagrippen 35 auf, und zwar mehrere etwa gleichmäßig über den Umfang des Pfannenkörpers 10 verteilt. Die Einschlagrippen sind radial außenseitig mit messerartigen Schneiden versehen. Diese Einschlagrippen ermöglichen eine noch stabilere Verankerung des Pfannenkörpers im Beckenknochen. Sie können sich bei Bedarf auch bis auf die Überdachung 28 erstrecken.

Der Pfannenkörper 10 kann im kranialen und/oder kaudalen Bereich noch Löcher für den Durchtritt von Knochenschrauben aufweisen, wobei die Schraubenlöcher vorzugsweise sanduhr- bzw. venturirohrartig ausgebildet sind, so daß die Schrauben zwängungsfrei unter unterschiedlichen Winkeln eingeschraubt werden können. Bei Bedarf können die Schraubenlöcher durch zugeordnete Pfropfen, Schrauben oder dergleichen verschließbar sein. Vorzugsweise erstrecken sich die Schraubenlöcher jeweils zwischen zwei benachbarten Einschlagrippen 35.

Die Fixierung des Pfanneneinsatzes 11 in der korrespondierenden Ausnehmung 13 des Pfannenkörpers 10 erfolgt vorzugsweise durch einen Schnappmechanismus, insbesondere einen in eine in der Pfannenkörper-Ausnehmung 13 ausgebildete Ringnut einrastenden Vorsprung, insbesondere Ringwulst oder Wulstabschnitt an der Außenseite des Pfanneneinsatzes 11. Alternativ kann der Ringvorsprung an der Innenseite der Ausnehmung 13 ausgebildet sein, der dann mit einer Ringnut am Außenumfang des Pfanneneinsatzes 11 korrespondiert. Grundsätzlich ist es auch denkbar, den Pfanneneinsatz 11 innerhalb der Ausnehmung 13 des Pfannenkörpers 10 durch einen gesonderten Federring zu halten, der innerhalb einer Ringnut nahe des oberen Eingangsrandes der Ausnehmung 13 plazierbar ist und den oberen Rand des Pfanneneinsatzes 11 überdeckt.

Die Oberfläche der im Pfanneneinsatz 11 ausgebildeten Höhlung 12 ist vorzugsweise mit einer Gleitschicht versehen, insbesondere einer Gleitschicht aus Metall, Keramik oder einem abriebfesten Kunststoff.

Zu den oben genannten Winkeln sei noch erwähnt, daß der Winkel "c" mindestens etwa 10 Grad beträgt. Die Winkel "a" und "b" betragen 0 bis 30, der Winkel "a" vorzugsweise etwa 12 bis 17 Grad, und der Winkel "b" etwa 8 bis 15 Grad.

Der Winkel "d" entspricht etwa dem Mittel aus den Winkeln "a" und "b".

Die Mittelpunkte der oben erwähnten Viertelkugelschalen 17, 18 sind in Figur 2 noch mit den Bezugsziffern 36, 37 gekennzeichnet. Der Abstand dieser beiden Mittelpunkte entspricht der Streckung des Pfannenkörpers in Richtung seiner Längsachse 15. Der Radius der Viertelkugelschalen 17, 18 ist in Figur 2 mit "R" angegeben. Der Winkel "c" ist ein Maß für die Biegung des länglichen Pfannenkörpers unter Ausbildung einer Nieren- bzw. Bohnenform in Abweichung von einer exakten Oval- bzw. Ellipsoidform.

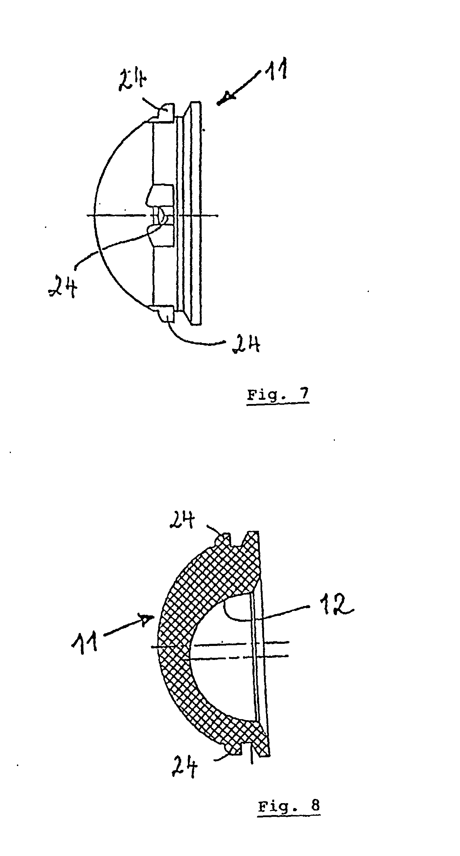

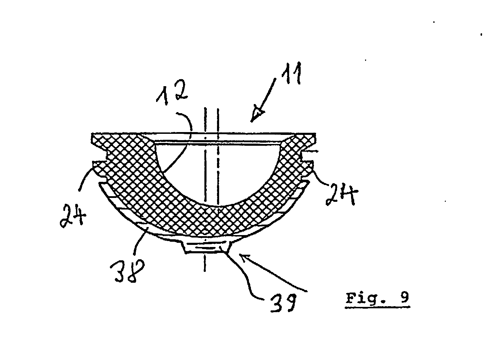

In Figur 9 ist ein Pfanneneinsatz 11 entsprechend dem in Figur 8 dargestellt. Der Pfanneneinsatz gemäß Figur 9 unterscheidet sich von dem gemäß Figur 8 dadurch, daß an seiner Rück- bzw. Außenseite ein schalenartiger metallischer Deckel 38 mit einem noppenartigen Verschlußelement in Form eines sich radial nach außen erstreckenden Vorsprungs 39 angeformt oder anderweitig befestigt ist. Dieser Deckel 38 ist eine Art "metalbacking". Er besteht vorzugsweise aus dem gleichen Material wie der zugeordnete Pfannenkörper 10. Der Vorsprung 39, der vorzugsweise konisch, d. h. als Kegelstumpf ausgebildet ist, verschließt im zusammengefügten Zustand von Pfanneneinsatz und Pfannenkörper einen korrespondierenden im Pfannenkörper ausgebildeten Durchgang. Der Pfanneneinsatz wird in den

Pfannenkörper eingerastet derart, daß der Vorsprung 39 einen dichten Abschluß des zugeordneten Durchgangs im Pfannenkörper garantiert. Die entsprechende polare Vorspannung auf den Vorsprung 39 erfolgt durch die erwähnte Verrastung des Pfanneneinsatzes längs der äquatorialen Peripherie im Pfannenkörper. Durch diese polar/äquatoriale Verklemmung bzw. Verrastung braucht die dazwischenliegende Titan- od. dgl. Schale 38 nicht an der Innenfläche des Pfannenkörpers 10 anzuliegen, sodaß in diesem Bereich kein Metallabrieb zu befürchten ist. Das "metalbacking" hat außerdem den Vorteil, daß im Bereich von Schraubenlöchern im Pfannenkörper 10 kein Fließen des Polyethylens des Pfanneneinsatzes 11 mehr zu befürchten ist.

### Bezugszeichenliste:

- 10: Pfannenkörper
- 11: Pfanneneinsatz (Inlay)
- 12: halbkugelige Höhlung
- 13: Ausnehmung
- 14: Umriß
- 15: Längsachse
- 16: Querachse
- 17: Viertelkugelschale
- 18: Viertelkugelschale
- 19: Verspannungs-Extrempunkt
- 20: Verspannungs-Extrempunkt
- 21: Verspannungs-Extrempunkt
- 22: Mittenachse
- 23: Eingangsebene
- 24: Vorsprung
- 25: Vertiefung
- 26: kranialer Bereich
- 27: kaudaler Bereich
- 28: Überdachung
- 29: Hauptpfannenebene
- 30: Bodenabflachung
- 31: Pfannenkörper-Boden
- 32: Durchgang
- 33: Deckel
- 34: Achse
- 35: Einschlagrippen
- 36: Kugelmittelpunkt
- 37: Kugelmittelpunkt
- 38: Deckel
- 39: noppenartiger Vorsprung
- c =: Winkel
- d =: Winkel
- a =: Winkel
- b: = Winkel

## Patentansprüche

1. Gelenkpfanne für eine Hüftgelenkendoprothese, mit einem länglich geformten Pfannenkörper (10) und einem Pfanneneinsatz (Inlay) (11), der auf der Frontseite eine halbkugelige Höhlung (12) zur Aufnahme einer Gelenkkugel aufweist,
**dadurch gekennzeichnet, daß**
der Umriß (14) des Pfannenkörpers (10) in Frontansicht zumindest in bezug auf die Längsachse (15) asymmetrisch ausgebildet ist.

2. Pfanne nach Anspruch 1,
**dadurch gekennzeichnet, daß**
der Pfannenkörper (10) in Frontansicht einen
- nieren- oder bohnenförmigen,
- bumerangartigen,
- trapezförmigen,
- dreieckförmigen,
- halbmondförmigen,
- herzförmigen,
- pfeilförmigen,
- halbkreisförmigen,
- oder dergleichen asymmetrischen, Umriß (14) aufweist.

3. Pfanne nach Anspruch 1 oder 2,
**dadurch gekennzeichnet, daß**
der Pfanneneinsatz (11) aus einem körperverträglichen Material, insbesondere Keramik oder Kunststoff, wie Polyethylen oder dergleichen, oder einer Kombination dieser Materialien besteht, während der Pfannenkörper (10) aus einem körperverträglichen Metall, insbesondere Kobaltchrommolybdänlegierung, Titan oder Titanlegierung, hergestellt ist.

4. Pfanne nach Anspruch 1, 2 oder 3,
**dadurch gekennzeichnet, daß**
die Rück- bzw. Außenseite des Pfanneneinsatzes (11) aus einem körperverträglichen Metall (Deckel 38, 39), insbesondere Kobaltchrommolybdänlegierung, Titan oder Titanlegierung, hergestellt ist, insbesondere in Zuordnung zu einem oder mehreren Durchgängen im Boden des Pfannenkörpers (10) bei etwaiger Ausbildung derartiger Durchgänge.

5. Pfanne nach einem der Ansprüche 1 bis 4,
**dadurch gekennzeichnet, daß**
die halbkugelige Höhlung (12) im Pfanneneinsatz (11) außermittig angeordnet ist.

6. Pfanne, insbesondere nach einem der Ansprüche 1 bis 5,
**dadurch gekennzeichnet, daß**
der Pfanneneinsatz (11) innerhalb einer korrespondierenden Ausnehmung (13) im Pfannenkörper (10) fixierbar ist, insbesondere in unterschiedlichen Winkelstellungen relativ zur Mittenachse (22) der Ausnehmung (13), wobei diese Ausnehmung (13) vorzugsweise konzentrisch im Pfannenkörper (10) ausgebildet ist.

7. Pfanne nach Anspruch 6,
**dadurch gekennzeichnet, daß**
der Pfanneneinsatz (11) eine rotationssymmetrische, insbesondere zylindrische, kegelstumpf- oder halbkugelförmige Schale ist, die Kraft- und/oder formschlüssig innerhalb der korrespondierenden Ausnehmung (13) im Pfannenkörper (10) fixierbar ist.

8. Pfanne nach Anspruch 2,
**dadurch gekennzeichnet, daß**
der Pfannenkörper (10) mit frontseitig nieren- oder bohnenförmigem Umriß (14) durch Verbindung bzw. Überbrückung von zwei zueinander im Winkel (c/2) relativ zur Querachse (16) des Pfannenkörpers (10) gegenüberliegend angeordneten Teilabschnitten zweier Rotationskörper, wie Kugel, Kegel, Zylinder oder dergleichen, insbesondere Viertelkugelschalen (17, 18) gebildet ist.

9. Pfanne nach einem der Ansprüche 1 bis 8,
**dadurch gekennzeichnet, daß**
die Frontseite des Pfannenkörpers (10) im kranialen Bereich (26) nach vorne überdacht (28) ist.

10. Pfanne nach einem der Ansprüche 1 bis 9,
**dadurch gekennzeichnet, daß**
die Frontseite des Pfannenkörpers (10) im kaudalen Bereich (27) nach innen bzw. hinten zurückgenommen ist.

11. Pfanne nach einem der Ansprüche 1 bis 10
**dadurch gekennzeichnet, daß**
der Boden (31) des Pfannenkörpers (10) einen Durchgang (32) aufweist, der durch einen Deckel, insbesondere verschiebbar oder drehbar gelagerten Deckel (33) verschließbar ist.

12. Pfanne nach Anspruch 11,
**dadurch gekennzeichnet, daß**
der Deckel an der Rück- bzw. Außenseite des Pfanneneinsatzes (11) angeordnet, insbesondere angeformt ist.

13. Pfanne nach einem der Ansprüche 1 bis 12,
**dadurch gekennzeichnet, daß**
der Pfannenkörper (10) außenseitig sich parallel zur Mittenachse (34) erstreckende Einschlagrippen (35) aufweist, insbesondere mehrere etwa gleichmäßig über den Umfang des Pfannenkörpers (10) verteilte Einschlagrippen mit messerartigen Schneiden.

14. Pfanne nach einem der Ansprüche 1 bis 13,
**dadurch gekennzeichnet, daß**
der Pfannenkörper (10) im kranialen und/oder kaudalen Bereich Löcher für den Durchtritt von Knochenschrauben aufweist, wobei die Schraubenlöcher vorzugsweise sanduhr- bzw. venturirohrartig ausgebildet sind, so daß die Schrauben zwängungsfrei unter unterschiedlichen Winkeln eingeschraubt werden können.

15. Pfanne nach Anspruch 14,
**dadurch gekennzeichnet, daß**
bei Bedarf die Schraubenlöcher durch zugeordnete Pfropfen, Schrauben oder dergleichen Verschlußelemente verschließbar sind.

16. Pfanne nach einem der Ansprüche 1 bis 15,
**dadurch gekennzeichnet, daß**
die Fixierung des Pfanneneinsatzes (11) in der korrespondierenden Ausnehmung (13) des Pfannenkörpers (10) durch einen Schnappmechanismus, insbesondere einen in eine in der Pfannenkörper-Ausnehmung (13) ausgebildete Ringnut einrastenden Vorsprung, insbesondere Ringwulst oder Wulstabschnitt an der Außenseite des Pfanneneinsatzes (11), erfolgt.

17. Pfanne nach einem der Ansprüche 1 bis 16,
**dadurch gekennzeichnet, daß**
die Oberfläche der im Pfanneneinsatz (11) ausgebildeten Höhlung (12) mit einer Gleitschicht versehen ist, insbesondere einer Gleitschicht aus Metall, Keramik oder einem abriebfesten Kunststoff.

18. Pfanne nach einem der Ansprüche 1 bis 17,
**dadurch gekennzeichnet, daß**
der Pfanneneinsatz (11) randseitig wenigstens einen sich radial nach außen erstreckenden Vorsprung (24) oder alternativ wenigstens eine Vertiefung aufweist, der bzw. die mit wenigstens einer in der Ausnehmung (13) des Pfannenkörpers (10) ausgebildeten Vertiefung (25) bzw. einem in der Ausnehmung (13) des Pfannenkörpers (10) ausgebildeten Vorsprung korrespondiert.

## Claims

1. An articular socket for a hip joint endoprosthesis, including an oblong-shaped socket body (10) and a socket insert (inlay) (11) having on the front side a semi-spherical cavity (12) for receiving an articular ball,
**characterized in that** the contour (14) of the socket body (10), in the front view, is configured asymmetrically at least relative to the longitudinal axis (15).

2. The socket according to claim 1,
**characterized in that** the socket body (10), in the front view, has a
- kidney-shaped or bean-shaped,
- boomerang-type,
- trapezoidal,
- triangular-shaped,
- crescent-shaped,
- heart-shaped,
- arrow-shaped,
- semicircular,
- or similar asymmetrical contour (14).

3. The socket according to claim 1 or 2,
**characterized in that** the socket insert (11) consists of a bodily tolerated material, in particular ceramics or synthetic material, such as polyethylene and similar, or of a combination of said materials, while the socket body (10) consists of a bodily tolerated metal, in particular cobalt chrome-molybdenum alloy, titanium or a titanium alloy.

4. The socket according to claim 1, 2 or 3,
**characterized in that** the rear side or outer side of the socket insert (11) is made of a bodily tolerated metal (cover 38, 39), in particular cobalt chrome-molybdenum alloy, titanium or a titanium alloy, in particular in association to one or more passages in the bottom of the socket body (10) with any such passages being formed.

5. The socket according to any one of claims 1 through 4,
**characterized in that** the semi-spherical cavity (12) in the socket insert (11) is arranged eccentrically.

6. The socket according to any one of claims 1 through 5,
**characterized in that** the socket insert (11) can be attached within a corresponding recess (13) in the socket body (10), in particular in different angular attitudes relative to the center axis (22) of recess (13), said recess (13) preferably being configured concentrically in the socket body (10).

7. The socket according to claim 6,
**characterized in that** the socket insert (11) is a rotation-symmetrical, in particular cylindrical, truncated cone-shaped or semi-spherical bowl, which can be fixed within the corresponding recess (13) in the socket body (10) in a force-transmitting and form-fitting manner.

8. The socket according to claim 7,
**characterized in that** the socket body (10) having the kidney-shaped or bean-shaped front side contour (14) is formed by joining or bridging two portions mutually arranged opposing each other in an angle (c/2) relative to the transverse axis (16) of the socket body (10) of two rotational bodies, such as a ball, cone, cylinder or similar, in particular quadrant bowls (17, 18).

9. The socket according to any one of claims 1 through 8,
**characterized in that** the front side of the socket body (10) in the cranial zone (26) is roofed over (28) towards the front.

10. The socket according to any one of claims 1 through 9,
**characterized in that** the front side of the socket body (10) in the caudal zone (27) is recessed towards the inside and towards the rear side, respectively.

11. The socket according to any one of claims 1 through 10,
**characterized in that** the bottom (31) of the socket body (10) has a passage (32) that can be closed by a cover, in particular a cover (33) mounted displaceable or rotatable.

12. The socket according to claim 11,
**characterized in that** the cover is arranged, in particular integrally formed on the rear side or outer side of the socket insert (11).

13. The socket according to any one of claims 1 through 12,
**characterized in that** the socket body (10) on the outer side has locking rips (35) extending in parallel to the center axis (34), in particular several locking rips distributed approximately uniformly over the circumference of the socket body (10) and having knife-like cutting edges.

14. The socket according to any one of claims 1 through 13,
**characterized in that** the socket body (10) in the cranial and/or caudal zone has holes for the passage of bone screws, with the screw holes being preferably configured hour glass-shaped or Venturi tube-shaped, so that the screws can be screwed in free from constraint under various angles.

15. The socket according to claim 14,
**characterized in that** the screw holes, if required, can be closed by associated plugs, screws or similar sealing elements.

16. The socket according to any one of claims 1 through 15,
**characterized in that** the fixation of the socket insert (11) in the corresponding recess (13) of the socket body (10) ensues by means of a snap mechanism, in particular a projection catching in a ring groove formed in the socket body recess (13), in particular a ring bead or bead section on the outer side of the socket insert (11).

17. The socket according to any one of claims 1 through 16,
**characterized in that** the surface of the cavity (12) formed in the socket insert (11) is provided with a sliding layer, in particular a sliding layer of metal, ceramics or an abrasion-resistant synthetic material.

18. The socket according to any one of claims 1 through 17,
**characterized in that** the socket insert (11), on the edge side, has at least one projection (24) extending radially towards the outer side, or, alternatively, has at least a depression, which projection or depression is in correspondence with at least one depression (25) formed in the recess (13) of the socket body (10) or with a projection formed in the recess (13) of the socket body (10).

## Revendications

1. Cavité articulaire pour une prothèse d'articulation de la hanche, avec un corps de cavité (10) formé en longueur et un insert de cavité (inlay) (11) qui, sur le côté avant présente un évidement en demi-sphère (12) pour recevoir une boule d'articulation,
**caractérisée en ce que**
le contour (14) du corps de cavité (10), vu de face, est, au moins en se référant à l'axe dans le sens de la longueur (15), formé de manière asymétrique.

2. Cavité articulaire selon la revendication 1,
**caractérisée en ce que**
le corps de cavité (10), vu de face, présente un contour (14)
- en forme de rein ou de haricot,
- de type "boomerang",
- en forme de trapèze,
- en forme de triangle,
- en forme de demi-lune,
- en forme de coeur,
- en forme de flèche,
- en forme de demi-cercle,
- ou d'un contour (14) asymétrique semblable.

3. Cavité articulaire selon la revendication 1 ou 2,
**caractérisée en ce que**
l'insert de cavité (11) se constitue d'un matériau supporté par le corps, en particulier de céramique ou d'un matériau synthétique tel du polyéthylène ou semblable, ou d'une combinaison de ces matériaux, alors que le corps de cavité (10) est fabriqué en un métal supporté par le corps, en particulier un alliage cobalt-chrome-molybdène, du titane ou un alliage de titane.

4. Cavité articulaire selon la revendication 1, 2 ou 3,
**caractérisée en ce que**
le côté dos ou extérieur de l'insert de cavité (11) est fabriqué en un métal supporté par le corps (couvercle 38, 39), en particulier un alliage cobalt-chrome-molybdène, du titane ou un alliage de titane, en particulier en affectation à une ou plusieurs ou plusieurs traversées dans le fond du corps de cavité (10) lors de l'éventuel formage de telles traversées.

5. Cavité articulaire selon l'une des revendications 1 à 4,
**caractérisée en ce que** l'évidement en demi-sphère (12) de l'insert de cavité (11) est disposé de manière excentrique.

6. Cavité articulaire en particulier selon l'une des revendications 1 à 5,
**caractérisée en ce que**
l'insert de cavité (11) peut se fixer dans un creux (13) correspondant du corps de cavité (10), en particulier selon plusieurs positions angulaires par rapport à l'axe central (22) du creux (13), ce creux (13) étant formé de manière préférée concentriquement dans le corps de cavité (10).

7. Cavité articulaire selon la revendication 6,
**caractérisée en ce que**
l'insert de cavité (11) est une coquille à symétrie de révolution, en particulier cylindrique, en tronc de cône ou en demi-sphère, pouvant se fixer en force et/ou par engagement dans le creux (13) correspondant dans le corps de cavité (10).

8. Cavité articulaire selon la revendication 2,
**caractérisée en ce que**
le corps de cavité (10) d'un contour (14) côté avant en forme de rein ou de haricot est formé par liaison ou pontage de deux sections partielles d'un corps de révolution, tel une boule, un cône, un cylindre ou semblables, disposées face à face en formant un angle (c/2) par rapport à l'axe transversal (16) du corps de cavité (10), en particulier de coquilles en quart de sphère (17, 18).

9. Cavité articulaire selon l'une des revendications 1 à 8,
**caractérisée en ce que**
le côté avant du corps de cavité (10), dans la zone crâniale (26), est se prolonge dans un toit vers l'avent (28).

10. Cavité articulaire selon l'une des revendications 1 à 9,
**caractérisée en ce que**
le côté avant du corps de cavité (10) dans la zone caudale (27) est reprise vers l'intérieur ou l'arrière.

11. Cavité articulaire selon l'une des revendications 1 à 10,
**caractérisée en ce que**
le fond (31) du corps de cavité (10) présente une traversée (32) obturable par un couvercle, en particulier un couvercle (33) coulissant ou logé de manière rotative.

12. Cavité articulaire selon la revendication 11,
**caractérisée en ce que**
le couvercle est disposé, en particulier formé intégralement, au côté de dos ou extérieur de l'insert de cavité (11).

13. Cavité articulaire selon l'une des revendications 1 à 12,
**caractérisée en ce que**
le corps de cavité (10) présente côté extérieur des nervures de verrouillage (35) s'étendant parallèlement à l'axe central (34), en particulier plusieurs nervures de verrouillage réparties sensiblement régulièrement sur le pourtour du corps de cavité (10) avec des tranchants de type "couteau".

14. Cavité articulaire selon l'une des revendications 1 à 13,
**caractérisée en ce que**
le corps de cavité (10) dans la zone crâniale et/ou caudale présente des trous pour la traversée de vis à os, les trous pour vis ayant, de manière préférée, une mise en forme de type "sablier" ou "tuyau venturi", de façon à ce que les vis puissent, sans forcer, être vissée selon plusieurs angles.

15. Cavité articulaire selon la revendication 14,
**caractérisée en ce que**
si nécessaire, les trous pour vis puissent s'obturer au moyen d'un bourrage, de vis ou d'éléments de fermeture similaires qui lui sont affectés.

16. Cavité articulaire selon l'une des revendications 1 à 15,
**caractérisée en ce que**
la fixation de l'insert de cavité (11) dans le creux (13) correspondant du corps de cavité (10) s'effectue au moyen d'un mécanisme d'encliquetage, en particulier d'une avancée, en particulier un bourrelet annulaire ou une section de bourrelet au côté extérieur de l'insert de cavité (11), qui s'encliquette dans une rainure annulaire formée dans le creux (13) de corps de cavité.

17. Cavité articulaire selon l'une des revendications 1 à 16,
**caractérisée en ce que**
la surface de l'évidement (12) formé dans l'insert de cavité (11) est pourvue d'une couche de glissement, en particulier d'une couche de glissement en métal, en céramique ou en une matière synthétique résistant à l'abrasion.

18. Cavité articulaire selon l'une des revendications 1 à 17,
**caractérisée en ce que**
l'insert de cavité (11), côté bord, présente au moins une avancée (24) s'étendant radialement vers l'extérieur, ou, de manière alternative, au moins un approfondissement, qui, l'un ou l'autre, corresponde au moins à un approfondissement (25) dans le creux (13) du corps de cavité (10) ou à une avancée formée dans le creux (13) du corps de cavité (10).
